# FASCICULE DE BREVET EUROPEEN

(11) **EP 1 368 081 B1**
(45) Date de publication et mention de la délivrance du brevet: **01.06.2005**
(21) Numéro de dépôt: 02714277.7
(22) Date de dépôt: 05.03.2002
(51) Int. Cl.: A61M 5/32, A61M 5/50

(54) **SERINGUE**
SPRITZE
SYRINGE

(30) Priorité: 05.03.2001 FR 0102986
(43) Date de publication de la demande: 10.12.2003
(73) Titulaire: Becton Dickinson France, 38800 Le Pont-de-Claix (FR)
(72) Inventeur: BARRELLE, Laurent, F-38250 Saint Nizier du Moucherotte (FR); JANSEN, Hubert, D-35041 Marburg-Michelbach (DE)
(74) Mandataire: Brédeville, Odile Marie
(86) Numéro de dépôt international: PCT/FR2002/000790
(87) Numéro de publication internationale: WO 2002/070054

(56) Documents cités:
- EP-A- 1 062 961
- WO-A-00/07646
- WO-A-00/56383
- WO-A-99/17823
- DE-A- 19 947 998
- US-A- 6 033 387

## Description

La présente invention concerne une seringue, ou dispositif similaire.

Une seringue comprend parfois un corps de seringue devant être relié, du côté proximal, à une bague. Tel est en particulier le cas d'une seringue comprenant un étui de protection, engagé autour du corps de seringue, cet étui et ce corps étant mobiles l'un par rapport à l'autre entre une position d'utilisation du dispositif, dans laquelle l'aiguille de la seringue est exposée, et une position de sécurité, dans laquelle cette aiguille est entourée par l'étui de manière à prévenir tout risque de piqûre ou de coupure, et donc de contamination éventuelle, de l'utilisateur ; la bague précitée forme alors un moyen de déplacement dudit corps et dudit étui l'un par rapport à l'autre entre lesdites positions d'utilisation et de sécurité.

Une seringue existante, de ce type, comprend un corps de seringue qui présente une collerette proximale, la bague comprenant des moyens d'assemblage propres à coopérer avec cette collerette.

Parmi les seringues existantes, certaines présentent l'inconvénient important de ne pas rendre impossible, ou tout au moins très difficile, un démontage du corps de seringue et de la bague.

Les seringues existantes présentent également différents inconvénients liés soit à leur relative complexité de structure, soit à des problèmes de fabrication résultant des tolérances dimensionnelles de leurs pièces constitutives ou des tolérances d'assemblage de ces pièces, soit à l'existence de jeux entre lesdites pièces.

Les documents WO 0 056 383 et DE 19 947 998 révèlent des dispositifs conformes au préambule de la revendication 1.

La présente invention vise à remédier à l'ensemble des inconvénients, cités ci-dessus.

Son objectif principal est donc de fournir une seringue rendant impossible, ou tout au moins très difficile, un démontage du corps de seringue et de la bague.

Un autre objectif de l'invention est de parvenir à l'objectif indiqué ci-dessus sans complexifier outre mesure la structure ou l'assemblage de la seringue, voire même en simplifiant cette structure et cet assemblage par rapport aux seringues connues.

La seringue concernée comprend un corps de seringue et une bague destinée à être assemblée au corps de seringue du côté proximal, le corps de seringue présentant à cet effet une collerette proximale et la bague comprenant des moyens d'assemblage propres à coopérer avec cette collerette.

Selon l'invention, lesdits moyens d'assemblage que comprend la bague comprennent :
- deux parties d'assemblage de forme courbe, disposées de manière à former substantiellement un anneau ; ces parties d'assemblage sont reliées à la bague au niveau de zones périphériques et sont réalisées en un matériau présentant un degré de souplesse élastique ; les zones périphériques de ces parties d'assemblage sont situées l'une par rapport à l'autre à une distance supérieure au diamètre exteme de ladite collerette tandis que les zones médianes de ces parties d'assemblage sont situées l'une par rapport à l'autre, à l'état non déformé de ces parties d'assemblage, à une distance inférieure au diamètre extérieur de la collerette ; ces zones médianes présentent, du côté de la bague par lequel le corps de seringue est engagé au travers des parties d'assemblage, des chanfreins conformés pour permettre l'effacement radial de ces zones médianes au moment de cet engagement, et
- des moyens distaux de butée, formant au moins une butée distale pour la collerette.

L'engagement de la collerette entre ces parties d'assemblage provoque la déformation élastique de celles-ci au niveau desdites zones médianes, dont les chanfreins permettent l'effacement radial. Une fois la collerette engagée au-delà de ces parties d'assemblage, le rappel élastique du matériau constituant ces parties d'assemblage réalise un verrouillage du corps de seringue.

Lesdites parties d'assemblage peuvent présenter des bossages de prise d'appui contre la collerette.

De préférence,
- lesdits des moyens distaux de butée comprennent deux parties de maintien en forme de fourche c'est-à-dire comprenant chacune une paire de bras et un corps de liaison reliant ces bras à la bague, les bras de chaque fourche étant conformés de manière à pouvoir entourer partiellement le corps de seringue ; et
- au moins une desdites parties de maintien présente une structure flexible, notamment au niveau de son corps de liaison, et est disposée de telle sorte que la distance séparant ses bras, d'une part, et lesdites parties d'assemblage, d'autre part, soit inférieure à l'épaisseur de la collerette.

Cette ou ces parties de maintien sont ainsi déformées lorsque la collerette est engagée entre elles et lesdites parties d'assemblage, et permettent de maintenir cette collerette plaquée contre ces parties d'assemblage. Une parfaite immobilisation axiale du corps de seringue est ainsi obtenue.

Selon une forme de réalisation préférée de l'invention, le corps de liaison de chaque partie de maintien présente une forme coudée, c'est-à-dire comprend une partie de base, reliée au reste de la bague et disposée sensiblement parallèlement à l'axe de cette bague, et une partie intermédiaire, reliée auxdits bras et disposée sensiblement perpendiculairement à l'axe de la bague.

Cette forme coudée favorise la déformabilité de la partie de maintien.

Avantageusement, au moins une des parties de maintien est conformée de telle sorte que ses bras sont, à l'état non déformé de la partie de maintien, inclinés en direction desdites parties d'assemblage.

De préférence, les parties de maintien sont diamétralement opposées.

Lesdits bras sont avantageusement réalisés en un matériau présentant un degré de souplesse élastique tel qu'ils peuvent être déformés élastiquement de manière à pouvoir recevoir entre eux, et enserrer, des corps de seringue de diamètres extérieurs différents.

Chacun des bras d'une partie de maintien peut comprendre au moins un bossage faisant saillie radialement vers l'intérieur.

Avantageusement, le dispositif comprend:
- un étui de protection, engagé autour du corps de seringue, cet étui et ce corps étant mobiles l'un par rapport à l'autre entre une position d'utilisation du dispositif, dans laquelle l'aiguille de la seringue est exposée, et une position de sécurité, dans laquelle cette aiguille est entourée par l'étui de manière à prévenir tout risque de piqûre ou de coupure, et donc de contamination éventuelle, de l'utilisateur ; l'étui de protection comprend au moins une lumière ou saignée longitudinale ;
- la bague forme un moyen de déplacement du corps de seringue et de l'étui de protection l'un par rapport à l'autre entre lesdites positions d'utilisation et de sécurité, et présente à cet effet une partie logée à l'intérieur de l'étui de protection, comportant des parties de maintien du corps de seringue, et au moins une partie de prise d'appui, engagée au travers de ladite lumière ou saignée et faisant saillie au-delà de l'étui de protection, qui sert à la prise d'appui de l'index ou du majeur de la main de l'utilisateur ;
- la tige du piston de la seringue comprend deux parties télescopiques, dont une, distale, est reliée au piston de la seringue, et dont l'autre, proximale, comporte une tête formant une partie de prise d'appui pour le pouce de l'utilisateur; et
- au moins un pont ruptible relie les deux parties télescopiques de la tige de piston, ce ou ces ponts étant propres à se rompre au-delà d'un seuil de forces antagonistes exercées sur ladite partie de prise d'appui et ladite tête, ce seuil étant supérieur à la force nécessaire au déplacement du piston mais inférieur aux forces antagonistes qu'un utilisateur est susceptible d'exercer manuellement sur ladite partie de prise d'appui et ladite tête.

La bague forme ainsi un moyen de déplacement du corps et de l'étui l'un par rapport à l'autre entre lesdites positions d'utilisation et de sécurité. L'action permettant d'amener le corps et l'étui en position de sécurité est réalisée par la même prise manuelle que la prise par laquelle la seringue est maintenue au cours de son utilisation, et peut donc être réalisée dans le prolongement de cette utilisation.

La rupture du ou des ponts ruptibles génére un mouvement relatif brusque du corps de seringue et de l'étui de protection, tel que ce corps de seringue et cet étui sont amenés en position de sécurité à l'issue de ce mouvement brusque. Ce mouvement limite ou même interdit tout contrôle intempestif du déplacement relatif du corps et de l'étui.

La bague peut comprendre des lumières pour le passage de l'étui et des épaulements de calage au niveau de ces lumières, permettant de caler en rotation la bague par rapport à l'étui.

Pour sa bonne compréhension, l'invention est à nouveau décrite ci-dessous en référence au dessin schématique annexé représentant, à titre d'exemple non limitatif, une forme de réalisation préférée de la seringue qu'elle concerne.
La figure 1 en est une vue en perspective, sous forme d'une seringue, avant injection du produit que contient cette seringue ;
la figure 2 en est une vue de côté, en coupe longitudinale ;
la figure 3 est une vue à échelle agrandie, d'une portion de la tige de piston que comprend la seringue ;
la figure 4 est une vue de détail d'un étui de protection que comprend la seringue, à échelle agrandie et en coupe longitudinale ;
la figure 5 est une vue de côté, à échelle agrandie, de cet étui et d'une bague d'actionnement que comprend la seringue ;
la figure 6 est une vue axiale de cet étui et de cette bague, du côté proximal de la seringue ;
la figure 7 est une vue en perspective de cette même bague ;
la figure 8 est une vue axiale de cette bague, et d'un corps de seringue assemblé à elle, du côté distal de la seringue ;
la figure 9 est une vue similaire à la figure 8, après mise en place dans la bague d'un corps de seringue de plus gros diamètre que le corps de seringue montré sur la figure 8 ;
la figure 10 est une vue de la seringue similaire à la figure 2, une fois l'injection réalisée ; et
la figure 11 est une vue de côté, à échelle agrandie, de la partie proximale de la seringue, une fois l'injection réalisée.

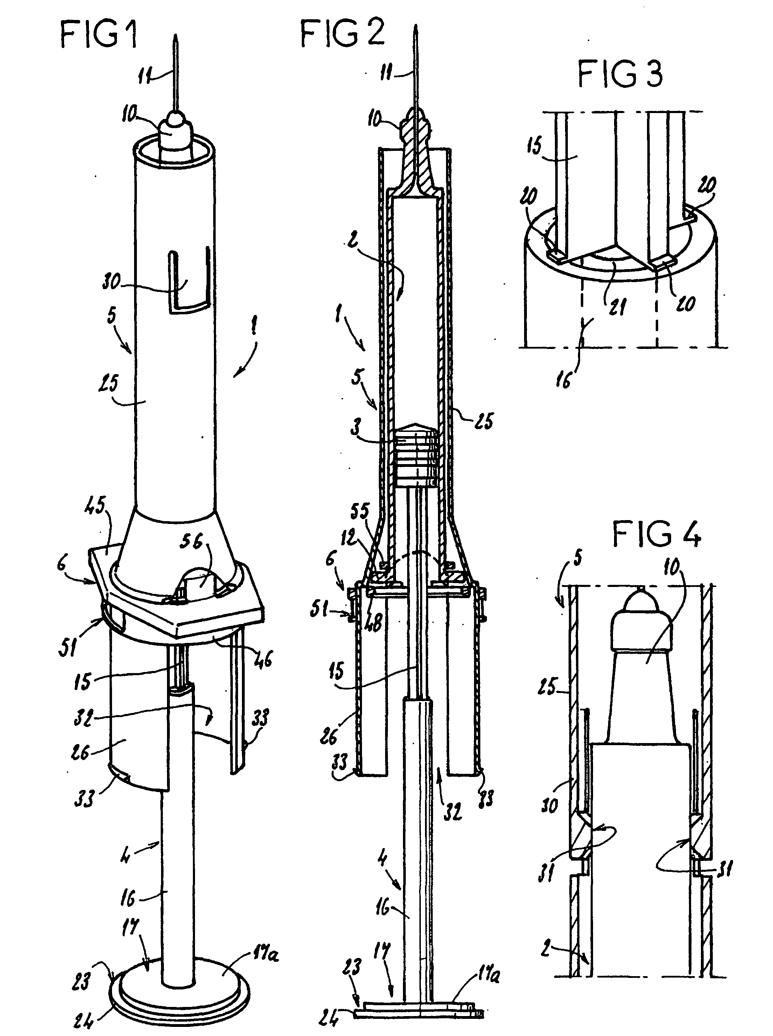

Les figures 1 et 2 représentent une seringue 1 à sécurité renforcée, comprenant un corps de seringue 2, un piston 3, une tige de piston 4, un étui de protection 5 et une bague 6 d'actionnement de l'étui 5.

Le corps de seringue 2 est de type classique, réalisé notamment en verre. II comprend un embout distal 10 comportant l'aiguille d'injection 11 et une collerette proximale 12, faisant saillie radialement vers l'extérieur, qui permet l'assemblage du corps 2 avec la bague 6, comme cela sera décrit plus loin. Le montage de l'aiguille 11 peut être réalisé autrement qu'au moyen d'un tel embout 10.

Le piston 3 est de type classique.

La tige de piston 4 comprend deux parties télescopiques 15, 16, dont celle distale 15 présente une section transversale en forme de croix et dont celle proximale 16 présente une forme tubulaire, propre à recevoir la partie 15 à coulissement. La partie 15 peut avoir une section d'une autre forme qu'en croix, ou la partie 15 peut présenter une forme tubulaire et la partie 16 présenter une section transversale en forme de croix.

La partie 16 comprend une tête d'appui 17 pour le pouce de l'utilisateur.

Comme le montre plus particulièrement la figure 3, chacune des quatre ailes que présente la partie 15 est reliée à la partie 16 par au moins un pont de matière 20, de sorte que ces parties 15 et 16 sont normalement maintenues dans une position d'extension, montrée sur les figures 1 et 2.

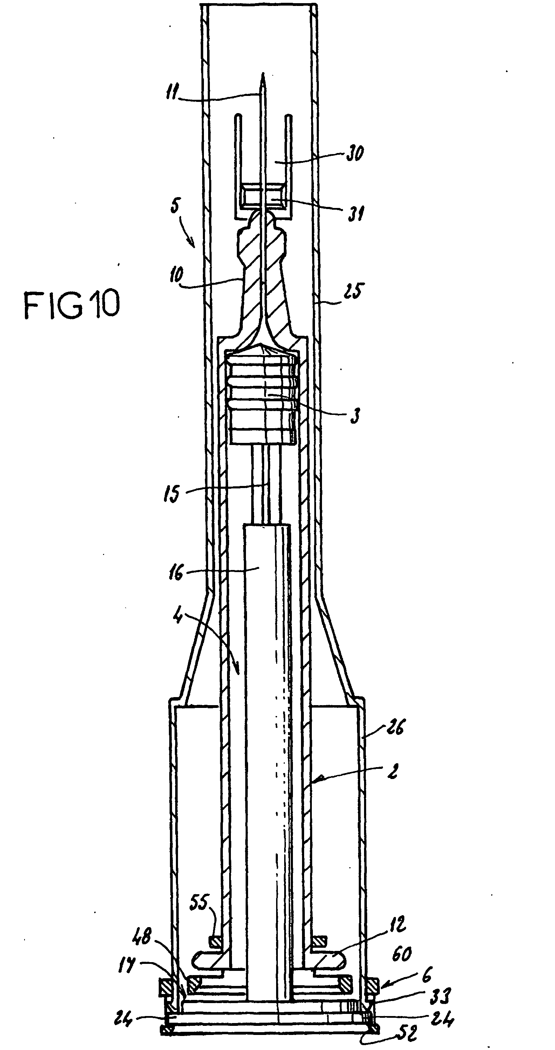

Ces ponts 20 sont suffisamment solides pour résister à la force qu'il est nécessaire d'exercer sur la tige 4 pour faire coulisser le piston 3 dans le corps 2, mais sont propres à se rompre au-delà d'un seuil de forces antagonistes susceptibles d'être exercées sur la bague 6 par l'index et le majeur de l'utilisateur, d'une part, et sur la tête 17 par le pouce de l'utilisateur, d'autre part, selon le geste classique d'actionnement d'une seringue. Une fois ces ponts 20 rompus, la partie 15 peut coulisser dans la partie 16 jusqu'à la position montrée sur la figure 10.

La partie 15 se prolonge, du côté proximal, au-delà des ponts 20, et cette prolongation 21 présente un diamètre extérieur légèrement inférieur au diamètre intérieur de la partie 16. Cette prolongation 21 permet de rigidifier longitudinalement la tige 4 afin d'éviter que cette tige présente, avant rupture des ponts 20, une zone de faiblesse dans le sens transversal.

La tête 17 présente une forme circulaire et comprend un redan périphérique 23, qui délimite une portion cylindrique 17a de la tête 17 et un rebord 24 faisant saillie radialement vers l'extérieur. Comme le montre la figure 11, le diamètre de cette portion cylindrique 17a est tel que cette portion cylindrique 17a peut être engagée avec ajustement dans la portion proximale 26 de l'étui 5 en fin d'injection.

L'étui 5 présente une portion distale 25 de diamètre interne supérieur au diamètre externe du corps 2 et une portion proximale 26 recevant la bague 6 à coulissement. L'ensemble de cet étui 5 est réalisé en une pièce par moulage d'un matériau synthétique présentant un degré de souplesse élastique telle qu'une matière plastique courante.

Comme le montrent les figures 1 et 4, la portion 25 présente deux fentes en forme de "U" diamétralement opposées, qui individualisent chacune une patte 30, cette patte 30 étant mobile radialement par déformation élastique au niveau de sa base. La face interne de chacune de ces pattes présente, du côté de l'extrémité de la patte 30 non reliée à l'étui 5, un bossage 31 faisant saillie à l'intérieur de cet étui.

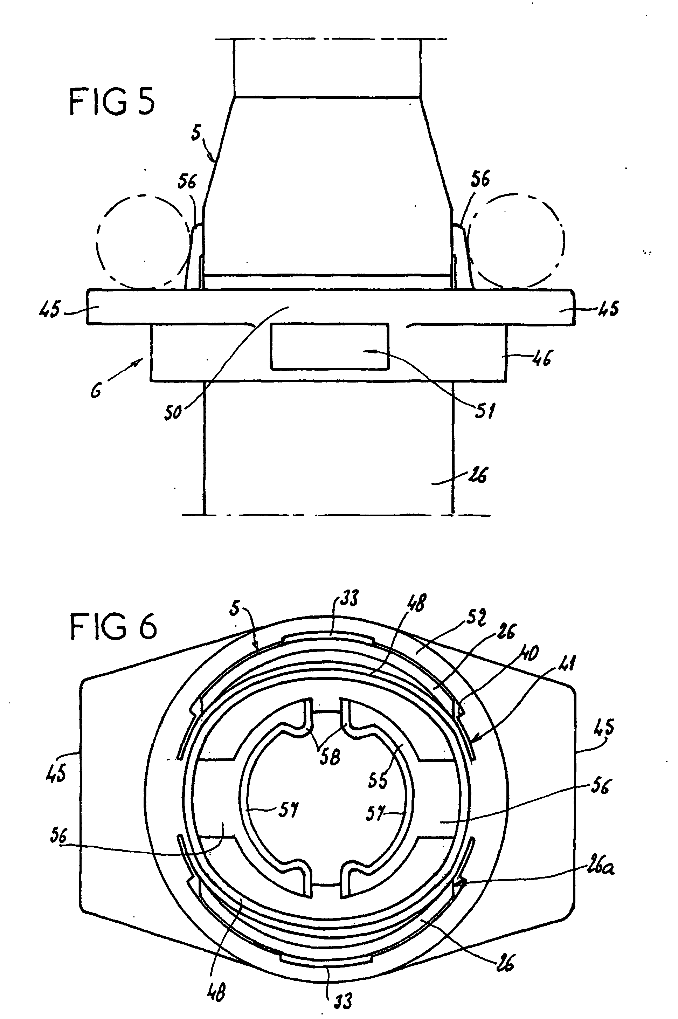

La portion 26 de l'étui présente deux méplats longitudinaux 26a (cf. figure 6) diamétralement opposés, au niveau desquels sont aménagés deux saignées 32 débouchant dans l'extrémité proximale de l'étui 5. Ces saignées 32 sont destinées à recevoir à coulissement des parties 56 de la bague 6, décrites plus loin.

Cette portion 26 présente en outre deux dents d'encliquetage 33 au niveau de son bord d'extrémité, à même d'assurer la rétention de la bague 6 sur l'étui 5, ainsi que cela sera également décrit plus loin.

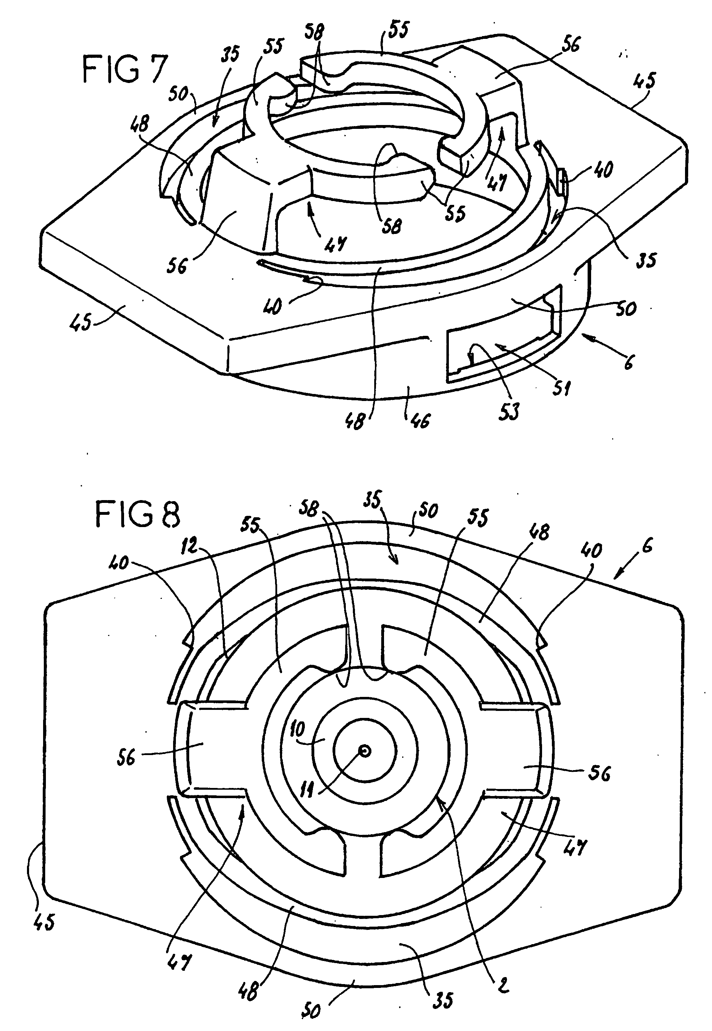

En référence à la figure 7, il apparaît que la bague 6 comprend deux lumières 35 de forme arquée, disposées symétriquement par rapport à l'axe de cette bague. Comme cela est visible sur la figure 6, ces lumières 35 sont destinées à recevoir les parties de l'étui 5 s'étendant entre lesdites saignées 32.

En retrait des extrémités longitudinales de ces lumières 35, sont formés des épaulements de calage 40, permettant de caler en rotation la bague 6 par rapport à l'étui 5. Chaque lumière 35 se prolonge au-delà de chaque épaulement 40, sous forme d'une fente 41.

La bague 6 présente deux ailettes d'appui 45 diamétralement opposées, faisant saillie radialement vers l'extérieur, une jupe proximale 46, deux parties de maintien 47 et deux parties d'assemblage 48. L'ensemble de cette bague 6 est réalisé en une pièce par moulage d'un matériau synthétique présentant un degré de souplesse élastique telle qu'une matière plastique courante.

Les ailettes 45 sont dimensionnées pour former des surfaces d'appui pour l'index et le majeur de l'utilisateur (schématisés par des cercles en traits interrompus sur la figure 5), et sont raccordées l'une à l'autre par des portions intermédiaires 50 de la bague 6.

La jupe 46 définit un logement propre à recevoir étroitement, en fin d'injection, la tête 17, comme le montrent les figures 10 et 11. Elle présente deux fenêtres 51 diamétralement opposées et un chanfrein proximal 52, visible notamment sur la figure 11.

Comme cela apparaît sur les figures 10 et 11, la bague 6 est destinée à être engagée sur l'extrémité proximale de l'étui 5 jusqu'au-delà des dents 33, ce qui la verrouille définitivement sur la portion 26 de l'étui 5. Le chanfrein 52 est quant à lui destiné à permettre l'engagement de la tête 17 dans le logement défini par la paroi 46, jusqu'à encliquetage, en fin d'utilisation, du rebord 24 derrière des nervures 53 aménagées sur les zones de la bague 6 qui délimitent les bords proximaux des fenêtres 51. Cet encliquetage assure le verrouillage de la tige 4 dans la position montrée sur les figures 10 et 11. La portion 17a de la tête 17 assure le maintien des dents 33 dans une position radiale extérieure, qui permet d'assurer le verrouillage de la bague 6.

Les parties de maintien 47 sont diamétralement opposées et présentent chacune une forme de fourche, c'est-à-dire comprennent chacune une paire de bras 55 et un corps de liaison 56 reliant ces bras 55 à la bague 6.

Les bras 55 sont conformés, notamment arrondis dans l'exemple représenté, de manière à pouvoir entourer partiellement le corps de seringue 2, et comprennent des chanfreins 57 aménagés sur leur côté proximal, par lequel un corps de seringue 2 est destiné à être introduit au travers de la bague 6.

Chaque bras 55 comprend également, au niveau de son extrémité libre, un bossage 58 faisant saillie radialement vers l'intérieur. Il peut comprendre plusieurs de tels bossages.

Le corps de liaison 56 de chaque partie de maintien 47 présente une forme coudée, c'est-à-dire comprend une partie de base, reliée au reste de la bague 6 et disposée sensiblement parallèlement à l'axe de cette bague 6, et une partie intermédiaire, reliée auxdits bras 55 et disposée sensiblement perpendiculairement à l'axe de la bague 6.

Les parties de base sont disposées sur le même diamètre que celui selon lequel font saillie les ailettes 45.

Ainsi que le montre la figure 5, les corps de liaison 56 sont conformés de telle sorte qu'après assemblage de la bague 6 sur l'étui 5, lesdites parties intermédiaires sont engagées à coulissement dans les saignées 32 et que lesdites parties de base font saillie par rapport à la paroi de l'étui 5, grâce aux méplats 26a de la portion 26.

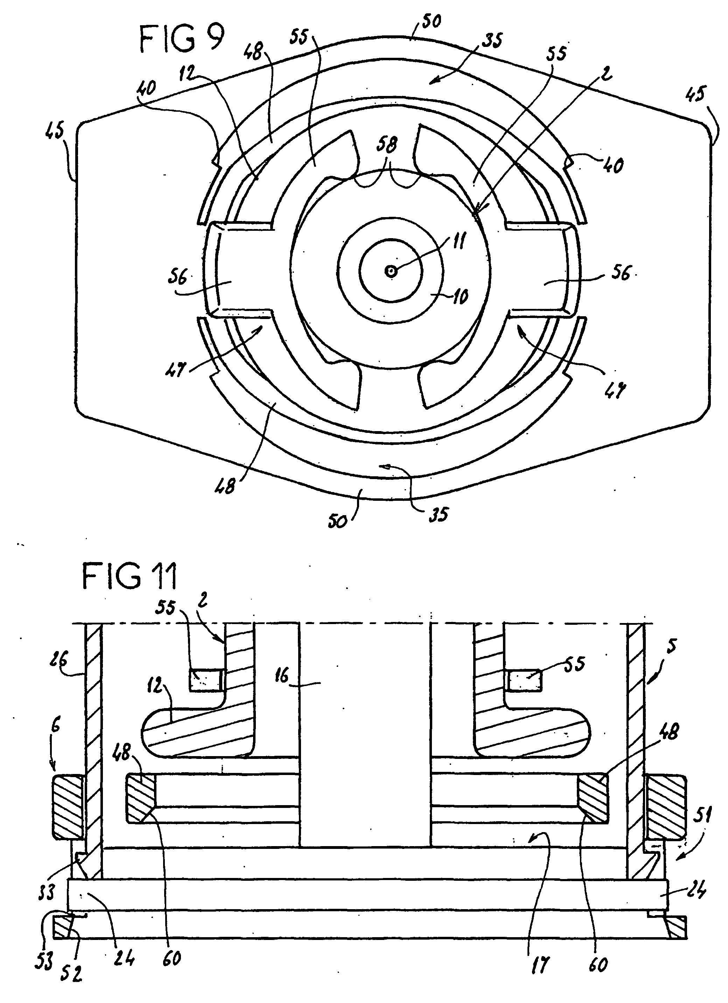

Comme cela se comprend en référence aux figures 8 et 9, les bras 55 sont destinés à recevoir entre eux et à enserrer le corps de seringue 2, et peuvent être déformés élastiquement de manière à pouvoir recevoir entre eux, et enserrer, des corps de seringue 2 de diamètres extérieurs différents, en particulier un corps 2 dit "0,5 ml" comme montré sur la figure 8 et un corps 2 dit "1 ml" comme montré sur la figure 9.

Les chanfreins 57 permettent de faciliter l'engagement d'un corps de seringue 2 entre ces bras, et les bossages 58 permettent d'assurer l'immobilisation et le centrage d'un corps de seringue 2 de diamètre réduit.

La relative souplesse des corps de liaison 56 dans le sens radial de la bague 6 permet de placer les bras 55 selon plusieurs positions radiales distinctes, et donc d'assurer un bon serrage d'un corps de seringue 2, sur une large gamme de diamètres de ceux-ci.

Les pattes 30 et les bossages 31 permettent quant à eux, ainsi que cela se déduit de la figure 4, de caler latéralement des corps de seringue 2 de diamètres extérieurs différents, les pattes 30 se déformant au besoin vers l'extérieur de manière à permettre l'effacement adéquat des bossages 31.

En outre, la présence desdites parties de base des corps de liaison 56 permet d'écarter les doigts de l'utilisateur par rapport à la paroi de l'étui 5, comme le montre la figure 5, éliminant ainsi tout risque de friction susceptible de freiner le mouvement d'activation du système de verrouillage, voire de compromettre ce verrouillage.

Les deux parties d'assemblage 48 sont délimitées par l'ouverture centrale de la bague 6 d'une part et par les lumières 35 d'autre part. Elles ont une épaisseur telle qu'elles sont déformables élastiquement dans le sens radial, cette déformabilité étant rendue importante grâce aux fentes 41.

Ces parties 48 constituent par exemple un anneau de forme ovale, relié, au niveau de leurs zones périphériques, les plus éloignées l'une de l'autre, à la bague 6, à proximité des parties de base des corps de liaison 56. Ces zones périphériques sont situées l'une par rapport à l'autre à une distance supérieure au diamètre externe de la collerette 12 du corps 2 tandis que les zones médianes de cet anneau, les plus proches l'une de l'autre, sont situées l'une par rapport à l'autre, à l'état non déformé de l'anneau, à une distance inférieure au diamètre extérieur de cette collerette 12.

La figure 11 montre que la partie proximale 26 de l'étui 5 présente un diamètre tel qu'il existe un espace entre la paroi de cette partie proximale 26 et lesdites zones médianes dudit anneau, cet espace permettant la déformation radiale de ces zones médianes.

En outre, des chanfreins 60 sont aménagés dans ces mêmes zones médianes.

Ainsi que cela se déduit des figures 6, 8, 9 et 11, la collerette 12 est destinée à être engagée au travers de l'anneau ovale précité jusqu'au-delà des parties 48. Les chanfreins 60 de ces parties 48 permettent l'effacement desdites zones médianes pour autoriser le passage de la collerette 12. Le rappel élastique de ces parties 48 dans leur forme d'origine permet le verrouillage de la collerette 12 au-delà de ces parties 48.

Une autre forme de réalisation peut consister en ce que les parties d'assemblage 48 constituent un anneau sensiblement circulaire avec un ou plusieurs bossages dans les zones médianes, les plus déformables.

Le corps 2 est alors parfaitement assemblé à la bague 6, sans aucun jeu radial et avec un jeu axial très limité.

En pratique, comme cela se comprend par comparaison des figures 2 et 10, l'utilisateur réalise l'injection en exerçant des pressions antagonistes sur les ailettes 45 et sur la tête 17, au moyen respectivement de son majeur et de son index, d'une part, et de son pouce, d'autre part, selon le geste classique précité.

A la fin de l'injection, le piston 3 vient en butée contre le fond du corps de seringue 2 ou la tête 17 vient en butée contre l'étui 5 ; lesdites forces antagonistes peuvent alors être exercées de manière plus intense, jusqu'à rupture des ponts 20. Il en résulte un déplacement relatif brusque de la bague 6, et donc du corps de seringue 2, par rapport à la tête 17, jusqu'à venue de la bague 6 et de la tête 17 dans une position de sécurité, montrée sur la figure 10, dans.laquelle l'aiguille 11 est complètement rétractée dans l'étui 5.

Ce mouvement brusque est réalisé dans le prolongement du geste d'utilisation de la seringue 1 pour l'injection. Le caractère brusque de ce mouvement, associé à l'écartement des doigts de l'utilisateur par rapport à l'étui 5 résultant de la présence desdites parties de base des corps de liaison 56, assure que la position de sécurité est effectivement atteinte dans tous les cas.

Le verrouillage de la tête 17 est réalisé automatiquement en fin de ce mouvement brusque, par engagement du rebord 24 derrière les nervures 53. L'engagement de la portion 17a de la tête 17 dans l'extrémité proximale de l'étui 5, comme le montre la figure 11, élimine, ou tout au moins rend très difficile, toute tentative de déverrouillage de la tige 4.

Ainsi qu'il apparaît de ce qui précède, l'invention foumit une seringue ayant pour avantages essentiels de rendre impossible, ou tout au moins très difficile, un démontage du corps de seringue et de la bague, et d'avoir des pièces constitutives assemblées sans aucun jeu entre elles, ou avec des jeux réduits, de manière à conférer une finition améliorée à cette seringue.

Il va de soi que l'invention n'est pas limitée à la forme de réalisation décrite ci-dessus à titre d'exemple mais qu'elle en embrasse au contraire toutes les variantes de réalisation couvertes par les revendications ci-annexées. Ainsi, invention peut se rapporter à un dispositif à usage médical, avec sécurité, comprenant
(a) un sous-ensemble du type seringue, comprenant un corps allongé selon un axe de référence; une aiguille disposée axialement, communiquant avec l'intérieur dudit corps, à l'extrémité distale de ce dernier ; un piston déplaçable à l'intérieur dudit corps jusqu'à une position distale en butée ; et une tige de piston émergeant à l'extrémité proximale dudit corps, avec une partie proximale de prise d'appui ;
(b) un sous-ensemble de sécurité, comprenant un étui de protection monté autour du corps, déplaçable axialement par rapport à ce demier entre deux positions, à savoir une position d'exposition dans laquelle l'aiguille est exposée et une position de sécurité dans laquelle l'aiguille est entièrement contenue à l'intérieur dudit étui de protection ;
selon l'invention, en combinaison :
(i) l'étui de protection est prolongé par une portion proximale au-delà de l'extrémité proximale du corps dans la position d'exposition dudit étui de protection, la tige de piston émergeant de l'extrémité proximale de ladite portion proximale ;
(ii) une bague d'actionnement s'étend radialement à partir de l'extrémité proximale du corps, en étant solidaire de cette dernière, et comporte une partie externe de prise d'appui, de part et d'autre du corps ;
(iii) des moyens de coulissement axial entre la bague d'actionnement et la portion proximale de i'étui de protection, ladite portion proximale étant montée librement en translation au travers de la bague ;
(iv) des moyens de rétraction axiale de la tige de piston sur elle-même, par poussée axiale sur la partie proximale de prise d'appui lorsque le piston est dans sa position distale en butée, en sorte que ladite partie proximale de prise d'appui vient en butée contre l'extrémité proximale de l'étui de protection, puis déplace ce demier vers sa position de sécurité, toujours par poussée.

## Revendications

1. Seringue comprenant un corps de seringue (2) et une bague (6) destinée à être assemblée au corps de seringue (2) du côté proximal, le corps de seringue (2) présentant à cet effet une collerette proximale (12) et la bague (6) comprenant des moyens d'assemblage (48) propres à coopérer avec cette collerette, lesdits moyens d'assemblage comprenant:
- deux parties d'assemblage (48) de forme courbe, disposées de manière à former substantiellement un anneau et reliées à la bague (6) au niveau de zones périphériques et
- des moyens distaux (47) de butée, formant au moins une butée distale pour la collerette (12);
**caractérisée en ce que** ces parties d'assemblage (48) sont réalisées en un matériau présentant un degré de souplesse élastique; les zones périphériques de ces parties d'assemblage (48) sont situées l'une par rapport à l'autre à une distance supérieure au diamètre externe de ladite collerette (12) tandis que les zones médianes de ces parties d'assemblage (48) sont situées l'une par rapport à l'autre, à l'état non déformé de ces parties d'assemblage (48), à une distance inférieure au diamètre extérieur de la collerette (12) ; ces zones médianes présentent, du côté de la bague (6) par lequel le corps de seringue (2) est engagé au travers des parties d'assemblage, des chanfreins (60) conformés pour permettre l'effacement radial de ces zones médianes au moment de cet engagement.

2. Seringue selon la revendication 1, **caractérisée en ce que** les parties d'assemblage (48) présentent des bossages de prise d'appui contre la collerette (12).

3. Seringue selon la revendication 1 ou la revendication 2, **caractérisée en ce que** :
- lesdits des moyens distaux (47) de butée comprennent deux parties de maintien (47) en forme de fourche c'est-à-dire comprenant chacune une paire de bras (55) et un corps de liaison (56) reliant ces bras (55) à la bague (6), les bras (55) de chaque fourche étant conformés de manière à pouvoir entourer partiellement le corps de seringue (2) ; et
- au moins une desdites parties de maintien (47) présente une structure flexible, notamment au niveau de son corps de liaison (56), et est disposée de telle sorte que la distance séparant ses bras (55), d'une part, et lesdites parties d'assemblage (48), d'autre part, soit inférieure à l'épaisseur de la collerette (12).

4. Seringue selon la revendication 3, **caractérisée en ce que** le corps de liaison (56) de chaque partie de maintien (47) présente une forme coudée, c'est-à-dire comprend une partie de base, reliée au reste de la bague (6) et disposée sensiblement parallèlement à l'axe de cette bague (6), et une partie intermédiaire, reliée auxdits bras (55) et disposée sensiblement perpendiculairement à l'axe de la bague (6).

5. Seringue selon la revendication 3 ou la revendication 4, **caractérisée en ce qu'**au moins une des parties de maintien (47) est conformée de telle sorte que ses bras (55) sont, à l'état non déformé de la partie de maintien (47), inclinés en direction desdites parties d'assemblage (48).

6. Seringue selon l'une des revendications 3 à 5, **caractérisée en ce que** les parties de maintien (47) sont diamétralement opposées.

7. Seringue selon l'une des revendications 3 à 6, **caractérisée en ce que** lesdits bras (55) sont réalisés en un matériau présentant un degré de souplesse élastique tel qu'ils peuvent être déformés élastiquement de manière à pouvoir recevoir entre eux, et enserrer, des corps de seringue (2) de diamètres extérieurs différents.

8. Seringue selon l'une des revendications 3 à 7, **caractérisée en ce que** chacun des bras (55) d'une partie de maintien (47) comprend au moins un bossage (58) faisant saillie radialement vers l'intérieur.

9. Seringue selon l'une des revendications 1 à 8, **caractérisée en ce qu'**elle comprend un étui de protection (5), engagé autour du corps de seringue (2), cet étui (5) et ce corps (2) étant mobiles l'un par rapport à l'autre entre une position d'utilisation de la seringue (1), dans laquelle l'aiguille (11) de la seringue est exposée, et une position de sécurité, dans laquelle cette aiguille (11) est entourée par l'étui (5) de manière à prévenir tout risque de piqûre ou de coupure, et donc de contamination éventuelle, de l'utilisateur ; l'étui de protection (5) comprend au moins une lumière ou saignée longitudinale (32);
- la bague (6) forme un moyen de déplacement dudit corps (2) et dudit étui (5) l'un par rapport à l'autre entre lesdites positions d'utilisation et de sécurité, et présente à cet effet une partie logée à l'intérieur de l'étui de protection (5), comportant des parties (47) de maintien du corps de seringue (2), et au moins une partie de prise d'appui (45), engagée au travers de ladite lumière ou saignée (32) et faisant saillie au-delà de l'étui de protection (5), qui sert à la prise d'appui de l'index ou du majeur de la main de l'utilisateur ;
- la tige de piston (4) de la seringue (1) comprend deux parties télescopiques (15,16), dont une, distale, est reliée au piston (3) de la seringue, et dont l'autre, proximale, comporte une tête (17) formant une partie de prise d'appui pour le pouce de l'utilisateur ; et
- au moins un pont ruptible (20) relie les deux parties télescopiques (15, 16) de la tige de piston (4), ce ou ces ponts (20) étant propres à se rompre au-delà d'un seuil de forces antagonistes exercées sur ladite partie de prise d'appui (45) et ladite tête (17), ce seuil étant supérieur à la force nécessaire au déplacement du piston (3) mais inférieur aux forces antagonistes qu'un utilisateur est susceptible d'exercer manuellement sur ladite partie de prise d'appui (45) et ladite tête (17).

10. Seringue selon la revendication 9, **caractérisée en ce que** la bague (6) comprend des lumières (35) pour le passage de l'étui (5) et **en ce qu'**elle comprend des épaulements de calage (40) au niveau de ces lumières (35), permettant de caler en rotation la bague (6) par rapport à l'étui (5).

11. Seringue selon la revendication 10, **caractérisée en ce que** chaque lumière (35) se prolonge au-delà d'un épaulement (40), sous forme d'une fente (41), pour conférer une flexibilité augmentée à la partie d'assemblage (48) correspondante.

12. Dispositif à usage médical, avec sécurité, comprenant
(a) un sous-ensemble du type seringue, selon l'une des revendications 1 à 11, comprenant un corps (2) allongé selon un axe de référence ; une aiguille (11) disposée axialement, communiquant avec l'intérieur dudit corps, à l'extrémité distale de ce dernier ; un piston (3) déplaçable à l'intérieur dudit corps jusqu'à une position distale en butée ; et une tige (4) de piston émergeant à l'extrémité proximale dudit corps (2), avec une partie proximale de prise d'appui (17);
(b) un sous-ensemble de sécurité, comprenant un étui de protection (5) monté autour du corps (2), déplaçable axialement par rapport à ce dernier entre deux positions, à savoir une position d'exposition (cf. figure 2) dans laquelle l'aiguille (11) est exposée et une position de sécurité (cf. figure 10) dans laquelle l'aiguille (11) est entièrement contenue à l'intérieur dudit étui de protection (11) ;
dispositif **caractérisé en ce que**, en combinaison :
(i) l'étui de protection (5) est prolongé par une portion proximale (26) au-delà de l'extrémité proximale du corps (2) dans la position d'exposition dudit étui de protection, la tige (4) de piston émergeant de l'extrémité proximale de ladite portion proximale (26) ;
(ii) la bague d'actionnement (6) s'étend radialement à partir de l'extrémité proximale du corps (2), en étant solidaire de cette dernière, et comporte une partie externe (45) de prise d'appui, de part et d'autre du corps (2) ;
(iii) des moyens (41) de coulissement axial entre la bague d'actionnement (6) et la portion proximale (26) de l'étui de protection (5), ladite portion proximale étant montée librement en translation au travers de la bague (6) ;
(iv) des moyens (20) de rétraction axiale de la tige (4) de piston sur elle-même, par poussée axiale sur la partie proximale de prise d'appui (17) lorsque le piston (3) est dans sa position distale en butée, en sorte que ladite partie proximale de prise d'appui (17) vient en butée contre l'extrémité proximale de l'étui de protection (5), puis déplace ce dernier vers sa position de sécurité, toujours par poussée.

## Patentansprüche

1. Spritze, die einen Spritzenkörper (2) und eine Ringstruktur (6) aufweist, die dazu vorgesehen ist, um mit dem Spritzenkörper (2) proximal verbunden zu werden, wobei der Spritzenkörper (2) zu diesem Zweck proximal einen Flansch (12) aufweist und die Ringstruktur Verbindungsmittel (48) aufweist, die mit dem Flansch wechselwirken können, wobei die Verbindungsmittel Folgendes aufweisen, nämlich
- zwei Verbindungsteile (48) mit gekrümmter Form, die derart angeordnet sind, um im Wesentlichen einen Ring zu bilden, und mit der Ringstruktur (6) in den peripheren Bereichen verbunden sind, und
- Abstandsmittel (47) mit Anschlag, die für den Flansch (12) zumindest einen distalen Anschlag bilden,
**dadurch gekennzeichnet, dass** die Verbindungsteile (48) ein Material aufweisen, das ein Maß an elastischer Flexibilität hat, wobei die Umfangsbereiche der Verbindungsteile (48) von einer Seite zur anderen einen Abstand haben, der größer ist als der Außendurchmesser des Flansches (12), wohingegen die mittleren Bereiche der Verbindungsteile (48) von einer Seite zur anderen bei nicht-deformiertem.Zustand der Verbindungsteile (48) einen Abstand haben, der kleiner ist als der Außendurchmesser des Flansches (12), wobei die mittleren Bereiche an der Seite der Ringstruktur (6), auf der der Spritzenkörper (2) in die Verbindungsteile hineingreift, Fasen (60) aufweisen, die derart ausgestaltet sind, um das radiale Zurückweichen dieser mittleren Bereiche im Augenblick des Eingreifens zu ermöglichen.

2. Spritze nach Anspruch 1, **dadurch gekennzeichnet, dass** die Verbindungsteile (48) den Flansch (12) stützende eingreifende Vorsprünge aufweisen.

3. Spritze nach Anspruch 1 oder Anspruch 2, **dadurch gekennzeichnet, dass**
- die Abstandsmittel (47) mit Anschlag zwei Halteteile (47) in Form einer Gabel, d.h. jeweils ein paar Arme (55) und ein Verbindungsstück (56), das die Arme (55) mit dem Ring (6) verbindet, aufweisen, wobei die Arme (55) einer jeden Gabel derart ausgebildet sind, dass diese den Spritzenkörper (2) teilweise umschließen können, und
- zumindest eines der Halteteile (47) eine flexible Struktur aufweist, insbesondere im Bereich seines Verbindungsstücks (56), und derart angeordnet ist, dass der Abstand, der einerseits die Gabeln (55) und andererseits die Verbindungsteile (48) voneinander trennt, kleiner ist als die Dicke des Flansches (12).

4. Spritze nach Anspruch 3, **dadurch gekennzeichnet, dass** das Verbindungsstück (56) eines jeden Halteteils (47) eine umgebogene Form, d.h. ein Basisteil aufweist, das mit dem Rest der Ringstruktur (6) verbunden ist und in etwa parallel zu der Achse der Ringstruktur (6) angeordnet ist, und ein Zwischenteil aufweist, das mit den Armen (55) verbunden ist und in etwa im rechten Winkel zu der Achse der Ringstruktur (6) angeordnet ist.

5. Spritze nach Anspruch 3 oder Anspruch 4, **dadurch gekennzeichnet, dass** zumindest eines der Halteteile (47) derart ausgebildet ist, dass die Arme (55) bei nichtdeformiertem Zustand der Halteteile (47) in die Richtung der Verbindungsteile (48) geneigt sind.

6. Spritze nach einem der Ansprüche 3 bis 5, **dadurch gekennzeichnet, dass** die Halteteile (47) diametral entgegengesetzt angeordnet sind.

7. Spritze nach einem der Ansprüche 3 bis 6, **dadurch gekennzeichnet, dass** die Arme (55) ein Material aufweisen, das ein Maß an elastischer Flexibilität hat, dass diese derart elastisch deformiert werden können, um dazwischen Spritzenkörper (2) von unterschiedlichen Außendurchmessern aufnehmen und umschließen zu können.

8. Spritze nach einem der Ansprüche 3 bis 7, **dadurch gekennzeichnet, dass** jeder Arm (55) eines Halteteils (47) zumindest einen Vorsprung (58) aufweist, der einen radial nach innen gerichteten Vorsprung ausbildet.

9. Spritze nach einem der Ansprüche 1 bis 8, **dadurch gekennzeichnet, dass** diese eine Schutzhülle (5) aufweist, die um den Spritzenkörper (2) herum eingreifend angeordnet ist, wobei die Hülle (5) und der Körper (2) gegeneinander von einer Position zur Verwendung der Spritze (1), in der die Nadel (11) der Spritze exponiert ist, in eine Sicherheitsposition, in der die Nadel (11) von der Hülle (5) derart umschlossen ist, dass jegliches Stech- oder Schneidrisiko und folglich jegliche eventuelle Kontamination des Benutzers verhindert wird, bewegt werden können, wobei die Schutzhülle (5) zumindest eine Längsöffnung oder einen Längseinschnitt (32) aufweist;
- die Ringstruktur (6) bildet ein Mittel zum Verschieben des Körpers (2) in Bezug auf die Hülle (5) von der Verwendungs- in die Sicherheitsposition und weist dazu ein im Inneren der Schutzhülle (5) angeordnetes Teil auf, das Teile (47) zum Halten des Spritzenkörpers (2) aufweist, und zumindest ein Haltegriffteil (45), das durch die Öffnung oder den Einschnitt (32) hindurchgreift und über die Schutzhülle (5) hinaus einen Vorsprung bildet, der dem Zeigefinger oder Mittelfinger der Hand des Benutzers als Haltegriff dient;
- die Kolbenstange (4) der Spritze (1) weist zwei Teleskopteile (15, 16) auf, von denen eines distal mit dem Kolben (3) der Spritze verbunden ist, und das andere proximal einen Kopf (17) aufweist, der ein Haltegriffteil für den Daumen des Benutzers ausbildet, und
- zumindest einen brechbaren Steg (20), der die beiden Teleskopteile (15, 16) der Kolbenstange (4) miteinander verbindet, wobei der oder die Stege (20) geeignet sind, um bei einer Gegenkraft, die über eine Schwelle hinausgeht, zu brechen, die auf das Haltegriffteil (45) und den Kopf (17) ausgeübt wird, wobei die Schwelle oberhalb der Kraft liegt, die zur Verschiebung des Kolbens (3) erforderlich ist, aber unterhalb der Gegenkräfte liegt, die von einer Bedienperson manuell auf das Haltegriffteil (45) und den Kopf (17) ausgeübt werden können.

10. Spritze nach Anspruch 9, **dadurch gekennzeichnet, dass** die Ringstruktur (6) Öffnungen (35) für das Hindurchtreten der Hülle (5) aufweist, und dass diese Klemmansätze (40) im Bereich der Öffnungen (35) aufweist, die ein Rotationsverklemmen der Ringstruktur (6) in Bezug auf die Hülle (5) ermöglichen.

11. Spritze nach Anspruch 10, **dadurch gekennzeichnet, dass** sich jede Öffnung (35) in Form eines Schlitzes (41) über einen Vorsprung (40) hinaus erstreckt, um dem entsprechenden Verbindungsteil (48) eine größere Flexibilität zu verleihen.

12. Vorrichtung zur sicheren medizinischen Verwendung, die Folgendes aufweist, nämlich
(a) ein Teilsystem vom Typ einer Spritze nach einem der Ansprüche 1 bis 11, das einen Körper (2) aufweist, der sich entlang einer Bezugsachse erstreckt; eine Spritze (11), die axial angeordnet ist und mit dem Inneren des Körpers an dessen distalem Ende in Verbindung steht; einen Kolben (3), der im Inneren des Körpers bis zu einer distalen Anschlagsposition verschiebbar ist; und eine Kolbenstange (4), die an dem proximalen Ende des Körpers (2) hervorsteht, mit einem proximalen Haltegriff (17);
(b) ein Sicherheitsteilsystem, das eine Schutzhülle (5) aufweist, die um den Körper (2) angeordnet ist, und axial in Bezug auf den Körper zwischen zwei Positionen verschiebbar ist, nämlich einer exponierten Position (vgl. Fig. 2), in der die Nadel (11) exponiert ist, und einer Sicherheitsposition (vgl. Fig. 10), in der die Nadel (11) vollständig im Inneren der Schutzhülle (11) vorliegt;
wobei die Vorrichtung **dadurch gekennzeichnet ist, dass** in Kombination
(i) die Schutzhülle (5) in der exponierten Position der Schutzhülle mit einem proximalen Teil (26) über das proximale Ende des Körpers (2) hinaus erstreckt vorliegt, wobei die Kolbenstange (4) über das proximale Ende des proximalen Teils (26) herausragt;
(ii) die Betätigungsringstruktur (6) sich ausgehend von dem proximalen Ende des Körpers (2) radial erstreckt, wobei diese mit dem letzteren verbunden ist, und auf beiden Seiten des Körpers (6) ein äußeres Haltegriffteil (45) aufweist;
(iii) Mittel (41) zum axialen Gleiten zwischen der Betätigungsringstruktur (6) und dem proximalen Teil (26) der Schutzhülle (5), wobei der proximale Teil durch ein Verschieben durch die Ringstruktur (6) hindurch frei aufgesetzt ist;
(iv) Mittel (20) zum axialen Zusammenschieben der Kolbenstange (4) durch axiales Drücken auf den proximalen Haltegriff (17), wenn sich der Kolben in seiner distalen Anschlagsposition befindet, derart, dass das proximale Haltegriffteil (17) gegen das proximale Ende der Schutzhülle (5) geschoben wird, dann Letztere durch steten Druck in Richtung ihrer Sicherheitsposition verschoben wird.

## Claims

1. A syringe comprising a syringe body (2) and a ring (6) which is intended to be joined to the syringe body (2) at the proximal end, the syringe body (2) having for this purpose a proximal flange (12), and the ring (6) comprising joining means (48) which are able to cooperate with this flange, said joining means comprising :
- two joining parts (48) of curved shape which are arranged in such a way as to substantially form an annulus and connected to the ring (6) in the area of peripheral zones and
- distal abutment means (47), forming at least one distal abutment for the flange (12), **characterized in that** these joining parts (48) are made of a material having a degree of elastic flexibility; the peripheral zones of these joining parts (48) are situated from one another at a distance greater than the external diameter of said flange (12), while the median zones of these joining parts (48), in the nondeformed state of these joining parts (48), are situated from one another at a distance smaller than the external diameter of the flange (12); these median zones have, on the side of the ring (6) via which the syringe body (2) is engaged through the joining parts, bevels (60) which are configured to permit radial clearance of these median zones during this engagement.

2. The syringe as claimed in claim 1, **characterized in that** the joining parts (48) have bosses for engagement against the flange (12).

3. The syringe as claimed in claim 1 or claim 2, **characterized in that** :
- said distal abutment means (47) comprise two fork-shaped holder parts (47), that is to say each comprising a pair of arms (55) and a connection body (56) connecting these arms (55) to the ring (6), the arms (55) of each fork being configured to be able to partially enclose the syringe body (2); and
- at least one of said holder parts (47) has a flexible structure, particularly in the area of its connection body (56), and is arranged in such a way that the distance separating its arms (55), on the one hand, and said joining parts (48), on the other, is less than the thickness of the flange (12).

4. The syringe as claimed in claim 3, **characterized in that** the connection body (56) of each holder part (47) has an elbowed configuration, that is to say comprises a base part connected to the rest of the ring (6) and arranged substantially parallel to the axis of this ring (6), and an intermediate part connected to said arms (55) and arranged substantially perpendicular to the axis of the ring (6).

5. The syringe as claimed in claim 3 or claim 4, **characterized in that** at least one of the holder parts (47) is configured in such a way that, in the nondeformed state of the holder part (47), its arms (55) are inclined in the direction of said joining parts (48).

6. The syringe as claimed in one of claims 3 through 5, **characterized in that** the holder parts (47) are diametrically opposed.

7. The syringe as claimed in one of claims 3 through 6, **characterized in that** said arms (55) are made of a material having a degree of elastic flexibility which is such that they can be elastically deformed in order to be able to receive between them, and tightly grip, syringe bodies (2) of different external diameters.

8. The syringe as claimed in one of claims 3 through 7, **characterized in that** each of the arms (55) of a holder part (47) comprises at least one boss (58) projecting radially inward.

9. The syringe as claimed in one of claims 1 through 8, **characterized in that** it comprises a protective casing (5) engaged around the syringe body (2), this casing (5) and this body (2) being movable relative to one another between a use position of the syringe (1), in which the needle (11) of the syringe is exposed, and a safety position, in which this needle (11) is surrounded by the casing (5), in such a way as to prevent any risk of stick injuries or cuts, and thus of possible contamination of the user; the protective casing (5) comprises at least one longitudinal slot or cutting (32);
- the ring (6) forms a means of displacing said body (2) and said casing (5) relative to one another between said use position and safety position, and for this purpose it has a part lodged inside the protective casing (5), including parts (47) for holding the syringe body (2), and at least one support part (45), engaged through said slot or cutting (32) and protruding beyond the protective casing (5), and which serves to support the index finger or middle finger of the user's hand;
- the plunger rod (4) of the syringe (1) comprises two telescopic parts (15, 16), one of which, the distal part, is connected to the plunger (3) of the syringe, and the other of which, the proximal part, has a head (17) forming a support part for the user's thumb; and
- at least one breakable bridge (20) connects the two telescopic parts (15, 16) of the plunger rod (4), said bridge or bridges (20) being able to break above a threshold of antagonistic forces exerted on said support part (45) and said head (17), this threshold being greater than the force needed to displace the plunger (3) but lower than the antagonistic forces which a user is likely to exert manually on said support part (45) and said head (17).

10. The syringe as claimed in claim 9, **characterized in that** the ring (6) comprises openings (35) for the passage of the casing (5), and wherein it comprises wedging shoulders (40) in the area of these openings (35), making it possible to wedge the ring (6) in terms of rotation relative to the casing (5).

11. The syringe as claimed in claim 10, **characterized in that** each opening (35) is continued beyond a shoulder (40) in the form of a slit (41) in order to give increased flexibility to the corresponding joining part (48).

12. Device for medical use, with safety comprising
(a) a subassembly of the syringe type as claimed in one of claims 1 through 11, comprising an elongate body (2) on a reference axis; a needle (11) arranged axially, communicating with the inside of said body, at the distal end thereof; a plunger (3) displaceable inside said body as far as a distal abutment position; and a plunger rod (4) emerging at the proximal end of said body (2), with a proximal support part (17);
(b) a safety subassembly, comprising a protective casing (5) mounted around the body (2), axially displaceable relative to the latter between two positions, namely an exposure position (cf. Figure 2) in which the needle (11) is exposed, and a safety position (cf. Figure 10) in which the needle (11) is entirely contained inside said protective casing (5);
device **characterized in that** in combination:
(i) the protective casing (5) is continued by a proximal portion (26) beyond the proximal end of the body (2) in the position of exposure of said protective casing, the plunger rod (4) emerging from the distal end of said proximal portion (26);
(ii) the actuating ring (6) extends radially from the proximal end of the body (2), being integral with the latter, and includes an outer support part (45) on either side of the body (2);
(iii) means (41) of axial sliding between the actuating ring (6) and the proximal portion (26) of the protective casing (5), said proximal portion being mounted freely in translation through the ring (6);
(iv) means (20) for axially retracting the plunger rod (4) on itself by means of an axial push on the proximal support part (17) when the plunger (3) is in its distal abutment position, so that said proximal support part (17) comes into abutment against the proximal end of the protective casing (5) then displaces the latter toward its safety position, again by pushing.
